# FASCICULE DE BREVET EUROPEEN

(11) **EP 0 793 492 B1**
(45) Date de publication et mention de la délivrance du brevet: **09.04.2003**
(21) Numéro de dépôt: 96901018.0
(22) Date de dépôt: 04.01.1996
(51) Int. Cl.: A61K 31/365, A61P 1/16, A61P 9/00, A61P 11/00, A61P 15/00, A61P 13/00, A61P 17/00, A61P 35/00, A61P 43/00

(54) **UTILISATION DE LA SCLAREOLIDE DANS LE TRAITEMENT D'AFFECTIONS CARACTERISEES PAR UNE PROLIFERATION EXCESSIVE DES CELLULES**
VERWENDUNG VON SKLAREOLIDE FÜR DIE BEHANDLUNG VON EXCESSIVEN ZELLWACHSTUMSKRANKHEITEN
USE OF SCLAREOLIDE FOR TREATING DISORDERS CHARACTERISED BY EXCESSIVE CELL PROLIFERATION

(30) Priorité: 04.01.1995 GB 9500024
(43) Date de publication de la demande: 10.09.1997
(73) Titulaire: SOCIETE DE CONSEILS DE RECHERCHES ET D'APPLICATIONS SCIENTIFIQUES (S.C.R.A.S.), 75016 Paris (FR)
(72) Inventeur: BRAQUET, Pierre, F-92380 Garches (FR); BIGG, Denis, F-91190 Gif-sur-Yvette (FR)
(74) Mandataire: Bourgouin, André
(86) Numéro de dépôt international: FR9600015
(87) Numéro de publication internationale: WO96020704

(56) Documents cités:
- CANCER LETT., vol. 21, no. 1, 1983, pages 29-35, XP002000469 H. OKAMOTO ET AL.: "Inhibition of 12-O-tetradecanoylphorbol-13-acetate-induc ed ornithine decarboxylase activity in mouse epidermis by sweetening agents and related compounds."

## Description

La (+)sclaréolide, [3aR-(3aα, 5aβ, 9aα, 9bα)]-décahydro-3a,6,6,9a-tetraméthylnaphto [2,1-b] furane-2(1H)-one est un terpénoïde bicyclique naturel que l'on trouve, par exemple, dans le tabac. Kaneko, H., Agr. Biol. Chem. 35(9) : 1461 (1971). La (+)sclaréolide a la structure suivante : 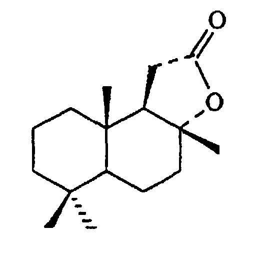 La (+)sclaréolide est connue pour augmenter ou développer les propriétés organoleptiques des produits alimentaires. Voir par exemple les brevets U.S. Nos. 4.917.913, 4.960.603, 4.966.783, 4.988.527 et 4.999.207. Ce composé a été utilisé comme parfum pour les cigarettes (brevet japonais No. 60/123,483) et comme additif pour éliminer le goût amer du café (brevet U.S. No. 4.988.532).

Cependant, à la connaissance de la déposante, la (+)sclaréolide n'a jamais été utilisée ou présentée comme un composé pharmacologiquement actif.

La présente invention concerne l'utilisation d'une quantité thérapeutiquement efficace de (+)sclaréolide pour la préparation d'un médicament pour inhiber une affection caractérisée par une prolifération excessive de cellules chez un patient (par exemple, un mammifère tel que l'homme).

Dans une configuration, le patient souffre d'une affection associée à une prolifération excessive de cellules bénigne (c'est-à-dire non maligne). Des exemples de telles affections sont la fibrose, l'hyperplasie prostatique bénigne, l'athérosclérose, la resténose, la glomérulosclérose, la chéloïde, le psoriasis et d'autres affections de la peau et maladies néo-plastiques non malignes.

Dans une autre configuration, le patient souffre d'une affection associée à une prolifération excessive de cellules maligne (par exemple le cancer). Des exemples de telles affections sont les adénomes, les carcinomes, les cancers constatés dans la prostate, le poumon, le foie, le pancréas, le cerveau, le sein et la peau, ainsi que la leucémie.

Une quantité thérapeutiquement efficace dépend de l'état traité et de la voie d'administration choisie, ainsi que de l'activité spécifique du composant utilisé et sera décidée finalement par le médecin ou le vétérinaire traitant. La (+)sclaréolide est administrée en une quantité de 0,1 à 500 mg/kg de poids corporel du patient (par exemple, 1 à 100 mg/kg de poids corporel du patient).

Alors qu'il est possible d'administrer la (+)sclaréolide sous la forme du composé pur ou substantiellement pur, ce produit peut aussi être présenté sous forme de formulation, de préparation ou de composition pharmaceutique. Les formulations à utiliser dans la présente invention, à la fois pour les êtres humains et les animaux, comprennent la (+)sclaréolide associée à un ou plusieurs support(s) pharmaceutiquement acceptable(s) de cette dernière et optionellement d'autres ingrédients thérapeutiques. Le support doit être "acceptable", c'est-à-dire compatible avec l'ingrédient (ou les ingrédients) actif(s) de la formulation et non nocif pour le sujet à traiter.

Les formulations peuvent être commodément présentées sous une forme à dosage unique et peuvent être préparées par l'une quelconque des méthodes bien connues dans l'art de la pharmacie. Toutes les méthodes comprennent la phase consistant à mettre la (+)sclaréolide en association avec un support qui peut contenir un ou plusieurs ingrédients accessoires. En général, les compositions destinées à la fabrication de comprimés (par exemple pour administration orale) ou de poudres, sont préparées par mélange uniforme et intime de la (+)sclaréolide avec des supports solides finement divisés, suivi si nécessaire, comme dans le cas des comprimés, d'une mise en forme du produit pour lui donner la forme et la dimension désirée.

Les compositions convenant pour l'administration parentérale (par exemple, sous-cutanée, intraveineuse ou intramusculaire), par ailleurs, comprennent commodément des solutions aqueuses stériles dans laquelle la (+)sclaréolide est soluble. De préférence, les solutions sont isotoniques avec le sang du sujet à traiter. Ces compositions peuvent être commodément préparées par dissolution de la (+)sclaréolide dans une solution aqueuse de ce genre, ladite solution étant ensuite rendue stérile. La composition peut être présentée en conteneurs à dose unique ou multiple, par exemple des ampoules ou des fioles scellées.

En conséquence, l'invention concerne également des compositions pharmaceutiques comprenant, comme principe actif, la (+)sclaréolide en association avec un ou plusieurs support(s) pharmaceutiquement acceptable(s).

Plus particulièrement, l'invention concerne l'utilisation de la (+)sclaréolide comme un médicament dans une méthode de traitement.

A moins qu'ils aient une autre définition, tous les termes techniques et scientifiques utilisés ici ont la même signification que celle couramment comprise par une personne de compétence ordinaire dans la technique à laquelle cette invention appartient.

### La(+)sclaréolide

La (+)sclaréolide est disponible auprès d'un certain nombre de sources du commerce, par exemple Aldrich Chemical Co., SL Louis, MO. La (+)sclaréolide peut également être préparée par synthèse, par exemple à partir de (-)sclareol (Aldrich Chem. Co.) ou d'acide homofarnésylique. Voir, par exemple, Coste-Maniere et ses collaborateurs, Tetrahedron Letters, 29(9) : 1017 (1988), Mantres et ses collaborateurs, Tetrahedron Letters 34(4) : 629 (1993) ; brevets allemands Nos. DE 4 301 555 et DE 3 942 358 ; et demande PCT No. WO 93/21174.

### Inhibition de la prolifération de l'hyperplasie prostatique bénigne (HPB)

Les solutions de (+)sclaréolide ont été préparées par dilution avec du diméthylsulfoxyde (DMSO) (0,5 %). Le milieu de culture utilisé était un milieu essentiel minimum (MEM, Gibco, Taisley, Royaume-Uni) sans aucun sérum mais auquel on avait ajouté de la L-glutamine (0,6 mg/ml, Gibco), de la gentamycine (40 µg/ml, Gibco), de la pénicilline (100 IV/ml, Gibco), et de la streptomycine (100 µg/ml ; Gibco). La thymidine tritiatée (dThd; spec. act., 48 Ci/mmole) a été achetée à Amersham (Little Chalfont, Royaume-Uni). Les solutions à ajouter au milieu d'incubation ont été préparées de manière extemporanée par dilution appropriée avec du MEM. Le tissu d'HPB a été obtenu de 10 patients (fourchette d'âge : 56-80 ans ; âge moyen ± déviation standard : 68 ± 12) possédant des prostates précédemment non traitées, qui avaient subi une prostatectomie tétropubique ouverte. Les spécimens ont été reçus frais de la salle d'opération dans du MEM maintenu à 4° C pendant 1-3 heures avant d'être traités pour culture d'organe tel que présenté en détail dans de Launoit Y. et ses collaborateurs, The Prostate, 13: 143 (1988). Pour chaque spécimen d'HPB, chaque condition expérimentale a été déterminée au moyen d'une série de 10 morceaux de tissu (0,5 - 1 mm³) placés dans une capsule de Pétri (3 cm de diamètre ; Gibco) contenant 2,5 ml de milieu MEM. Ces cultures ont été incubées dans un milieu témoin ou dans un milieu auquel on avait ajouté soit 10 nM de Facteur de Croissance Epithélial (FCE) soit 10 nM de Facteur de Croissance de Fibroblastes basique (FCFb), avec ou sans 10⁻⁴M (concentration finale) de (+)sclaréolide. Du FCE ou du FCFb ont été ajoutés au commencement de la culture. La (+)sclaréolide a été ajoutée 24 heures après le FCE ou le FCFb. Les cultures ont été immergées dans de l'EFA fixateur (éthanol 96° (70 % en volume), formol neutre (25 % en volume), acide acétique, (5 % en volume)) à la 72ème heure après le commencement de la culture, c'est-à-dire à la 48ème heure après l'addition de la (+)sclaréolide. De la thymidine tritiatée (2 µCi/ml MEM) a été ajoutée au milieu de culture 4 fois, à savoir, 36 heures, 24 heures, 12 heures et 1 heure avant fixation.

La méthode de marquage avec la thymidine tritiatée permet d'estimer les indices de marquage à la thymidine (IMT). L'IMT représente le pourcentage de cellules engagées dans la phase S du cycle de cellules. Cet indice représente donc une estimation indirecte de la vitesse de prolifération dans un tissu donné. La méthodologie utilisée ici est identique à celle présentée en détail dans de Launoit, Y. et ses collaborateurs, The Prostate, 13:143 (1988). L'IMT a été déterminé comme suit. On a coupé deux sections de chaque spécimen d'HPB. L'IMT a été déterminé séparément dans les compartiments stromal et épithélial de chaque HPB. Dans ce but, les comptes d'IMT ont été faits sur 300-800 cellules stromales et 300-800 cellules épithéliales par section d'HPB. Ainsi, au total, 600-1 600 cellules stromales et 600-1 600 cellules épithéliales ont été comptées par spécimen d'HPB et, au total, 6 000-16 000 cellules stromales et 6 000-16 000 cellules épithéliales par condition expérimentale. Le marquage nucléaire a été considéré comme positif lorsqu'un noyau a été couvert par un nombre de grains d'argent dont la valeur moyenne était 10 fois plus élevée que celle du fond.

Il a été constaté que la (+)sclaréolide inhibait la prolifération de cellules organotypiques de HPB stimulée par le FCFb à 70 % et inhibait la prolifération de cellules d'HPB stimulée par le Facteur de Croissance Epithélial à 60 % pour une concentration de 10⁴M.

### Inhibition de la prolifération de fibroblastes

On a utilisé une culture de cellules d'embryons de souris (CES) confluantes pour déterminer la prolifération de fibroblastes par mesure de l'incorporation de [³H] méthyle thymidine dans l'ADN de ces cellules. Les CES ont été mises en suspension dans un milieu essentiel modifié de Dulbecco (DMEM ; Gibco). Le DMEM dans cet essai contenait 1 g/l de glucose, 100 µg/ml de pénicilline, 100 µg/ml de streptomycine, et 10 % de sérum de veau foetal. Des plaques de culture à 24 cavités ont été remplies avec 500 µl de la suspension (70 000 cellules / cavité) et maintenues 24 heures dans un incubateur à 37° C et 5 % de CO₂.

Le jour suivant, le milieu de culture dans chaque cavité a été remplacé par 500 µl de DMEM contenant 0,5 % de sérum de veau foetal, en vue de réduire la vitesse de reproduction des cellules et de maintenir les cellules dans une phase stationnaire. Le jour suivant, le milieu de culture a été remplacé (1 µl pour chaque cavité) et les cellules ont été stimulées avec du FCFb avec ou sans une solution de concentrations diverses de (+)sclaréolide. Les solutions de (+)sclaréolide ont été préparées par dilution aqueuse avec du DMSO. Le jour suivant, de la [³H] méthyle thymidine (1 µCi/ml) a été ajoutée et les plaques de culture ont été maintenues pendant 4 heures à 37° C. Le milieu d'incubation a ensuite été enlevé et on a ajouté 1 ml d'acide trichloracétique à 10 % refroidi (ATC, Sigma Chemical). 30 minutes plus tard, à 4° C, l'ATC a été enlevé, les plaques de culture ont été rincées avec de l'eau et on a ajouté 500 µl de NaOH (0,3 m) à chaque cavité. Les plaques de culture ont été maintenues à 4° C pendant une nuit. Le jour suivant, les cultures de chaque cavité ont été transférées dans des fioles à scintillation. Le NaOH a été neutralisé avec 500 µl de HCl (0,3N), et la radioactivité des fioles de scintillation a été mesurée au moyen d'un compteur à scintillation Beckmann (modèle # L56000SG).

Il a été constaté que la (+)sclaréolide inhibait la prolifération de fibroblastes stimulée par le FCFb, à 50 % pour une concentration de 10⁻⁷M et à 70 % pour une concentration de 10⁻⁵M.

## Revendications

1. Composition pharmaceutique comprenant de la (+) sclaréolide, en association avec un ou plusieurs support(s) pharmaceutiquement acceptable(s).

2. Utilisation de la (+) sclaréolide pour la préparation d'un médicament pour le traitement de la fibrose, l'hyperplasie prostatique bénigne, l'athérosclérose, la resténose, la glomérulosclérose, la chéloïde, le psoriasis et les autres affections de la peau et les maladies néoplastiques non malignes, les adénomes, les carcinomes, les cancers constatés dans la prostate, le poumon, le foie, le pancréas, le cerveau, le sein et la peau, ainsi que la leucémie.

3. Utilisation de la (+) sclaréolide selon la revendication 2 pour la préparation d'un médicament destiné au traitement de l'hyperplasie prostatique bénigne ou de la fibrose.

4. Utilisation selon la revendication 2, **caractérisée en ce que** ladite (+) sclaréolide est administrée par voie parentérale.

5. Utilisation selon la revendication 4, **caractérisée en ce que** ladite (+) sclaréolide est administrée par voie sous-cutanée.

6. Utilisation selon la revendication 2, **caractérisée en ce que** ladite (+) sclaréolide est administrée par voie orale.

7. Utilisation selon la revendication 3, **caractérisée en ce que** ladite (+) sclaréolide est administrée par voie parentérale.

8. Utilisation selon la revendication 7, **caractérisée en ce que** ladite (+) sclaréolide est administrée par voie sous-cutanée.

9. Utilisation selon la revendication 3, **caractérisée en ce que** ladite (+) sclaréolide est administrée par voie orale.

## Claims

1. Pharmaceutical composition containing (+) sclareolide, in association with one or more pharmaceutically acceptable support(s).

2. Use of (+) sclareolide for the preparation of a medicament intended to treat fibrosis, benign prostate hyperplasia, atherosclerosis, restenosis, glomerulosclerosis, cheloid, psoriasis and other diseases of the skin and non-malignant neoplastic diseases, adenomas, carcinomas, cancers found in the prostate, the lungs, the liver, the pancreas, the brain, the breast and the skin, as well as leukemia.

3. Use of (+) sclareolide according to claim 2 for the preparation of a medicament intended to treat benign prostate hyperplasia or fibrosis.

4. Use according to claim 2, chararacterized in that said (+) sclareolide is administered by parenteral route.

5. Use according to claim 4, **characterized in that** said (+) sclareolide is administered by subcutaneous route.

6. Use according to claim 2, **characterized in that** said (+) sclareolide is administered orally.

7. Use according to claim 3, chararacterized in that said (+) sclareolide is administered by parenteral route.

8. Use according to claim 7, **characterized in that** said (+) sclareolide is administered by subcutaneous route.

9. Use according to claim 3, **characterized in that** said (+) sclareolide is administered orally.

## Patentansprüche

1. Pharmazeutische Zusammensetzung, die (+)Sclareolid in Kombination mit einem oder mehreren pharmazeutisch verträglichen Trägern enthält.

2. Verwendung von (+)Sclareolid für die Herstellung eines Medikaments für die Behandlung der Fibrose, der benignen Prostatahyperplasie, der Atherosklerose, der Restenose, der Glomerulosklerose, des Keloids, der Psoriasis und anderer Erkrankungen der Haut und nicht maligner neoplastischer Erkrankungen, von Adenomen, Karzinomen, Krebs, der in Prostata, Lunge, Leber, Pankreas, Gehirn, Brust und Haut festgestellt wurde, sowie von Leukämie.

3. Verwendung von (+)Sclareolid nach Anspruch 2, für die Herstellung eines Medikaments für die Behandlung der benignen Prostatahyperplasie oder der Fibrose.

4. Verwendung nach Anspruch 2, **dadurch gekennzeichnet, daß** das (+)Sclareolid auf parenteralem Weg verabreicht wird.

5. Verwendung nach Anspruch 4, **dadurch gekennzeichnet, daß** das (+)Sclareolid auf subkutanem Weg verabreicht wird.

6. Verwendung nach Anspruch 2, **dadurch gekennzeichnet, daß** das (+)Sclareolid auf oralem Weg verabreicht wird.

7. Verwendung nach Anspruch 3, **dadurch gekennzeichnet, daß** das (+)Sclareolid auf parenteralem Weg verabreicht wird.

8. Verwendung nach Anspruch 7, **dadurch gekennzeichnet, daß** das (+)Sclareolid auf subkutanem Weg verabreicht wird.

9. Verwendung nach Anspruch 3, **dadurch gekennzeichnet, daß** das (+)Sclareolid auf oralem Weg verabreicht wird.
